# EUROPEAN PATENT SPECIFICATION

(11) **EP 4 167 919 B1**
(45) Date of publication and mention of the grant of the patent: **26.11.2025**
(21) Application number: 21736383.7
(22) Date of filing: 22.06.2021
(51) Int. Cl.: A61K 31/197, A61H 9/00, A61P 25/14

(54) **ANTI-SPASMODIC COMPOSITIONS IN COMBINATION WITH NEGATIVE PRESSURE THERAPY FOR USE IN PREVENTING SPACTICITY**
ANTISPASTISCHE ZUSAMMENSETZUNG IN KOMBINATION MIT NEGATIVDRUCKTHERAPIE ZUR VERWENDUNG BEI DER VERHINDERUNG VON SPASTIZITÄT
COMPOSITIONS ANTISPASMODIQUES EN COMBINAISON AVEC UNE THÉRAPIE PAR PRESSION NÉGATIVE POUR UNE UTILISATION DANS LA PRÉVENTION DE LA SPASTICITÉ

(30) Priority: 22.06.2020 US 202063042246 P
(43) Date of publication of application: 26.04.2023
(73) Proprietor: Otivio AS, 0277 Oslo (NO)
(72) Inventor: MATHIESEN, Iacob, N-0277 Oslo (NO); INMAN, Laura Anne, N-0376 Oslo (NO)
(74) Representative: Inspicos P/S
(86) International application number: PCT/IB2021/055515
(87) International publication number: WO 2021/260556

(56) References cited:
- WO-A1-2019/064288
- CHANG ERIC ET AL: "A Review of Spasticity Treatments: Pharmacological and Interventional Approaches", CRITICAL REVIEWS IN PHYSICAL AND REHABILITATION MEDICINE, vol. 25, no. 1-2, 1 January 2013 (2013-01-01), US, pages 11 - 22, XP055839432, ISSN: 0896-2960, Retrieved from the Internet <URL:https://www.ncbi.nlm.nih.gov/pmc/articles/PMC4349402/pdf/nihms666511.pdf> DOI: 10.1615/CritRevPhysRehabilMed.2013007945
- SUNDBY ØYVIND HEIBERG ET AL: "The acute effects of lower limb intermittent negative pressure on foot macro- and microcirculation in patients with peripheral arterial disease", PLOS ONE, vol. 12, no. 6, 7 June 2017 (2017-06-07), pages 1 - 16, XP055839530, Retrieved from the Internet <URL:https://storage.googleapis.com/plos-corpus-prod/10.1371/journal.pone.0179001/1/pone.0179001.pdf?X-Goog-Algorithm=GOOG4-RSA-SHA256&X-Goog-Credential=wombat-sa@plos-prod.iam.gserviceaccount.com/20210909/auto/storage/goog4_request&X-Goog-Date=20210909T102511Z&X-Goog-Expires=86400&X-Goog-SignedHeaders=h> DOI: 10.1371/journal.pone.0179001

## Description

### FIELD OF THE DISCLOSURE

The disclosure relates generally to methods, compositions, and associated devices for treating neurodegenerative diseases and associated symptoms, including treatment of spasticity and/or prevention of spasticity and pain. The methods, compositions and devices may be configured to include application of negative pressure to a body of a patient in order to reverse spasticity and pain in the body and/or to prevent future occurrence of spasticity and pain in the body, as well as to improve neurological symptoms of neurodegenerative diseases.

### BACKGROUND

Spasticity is a common symptom of injuries or diseases affecting the central nervous system, including brain injury, spinal cord injury, cerebral palsy, stroke, fibromyalgia, myalgic encephalomyelitis/chronic fatigue syndrome, neuromyelitis optica, Parkinson's, amyotrophic lateral sclerosis, multiple sclerosis (MS) and additional neurodegenerative diseases. The term spasticity is generally used to refer to involuntary and sustained contractions of muscles, such as associated with muscle stiffness, cramps, exaggerated movement and reflexes, clonus, or spasms, and is a major cause of disability in individuals with impaired central nervous systems.

As a particular example, MS is a chronic disease of the nervous system commonly referred to as an immune-mediated disorder, in which the immune system is believed to incorrectly attack the central nervous system and may damage the protective myelin sheath surrounding nerve fibers, oligodendrocytes, and/or the underlying nerve fibers. The resulting demyelination, inflammation and damage to the nerve fibers can cause a variety of symptoms, including spasticity, fatigue, muscle weakness, vision problems, unsteady gait, numbness, tingling, dizziness, depression, pain and several others.

While the symptoms of MS are unpredictable and vary both from one patient to another and for a single patient over time, a majority of patients eventually develop a steady progression of symptoms. There is currently no recognized cure for MS, and known treatments are generally only directed to slowing the progression of the disease and/or worsening of symptoms.

Spasticity is one of the most prevalent and challenging of all MS symptoms and, while not completely understood, is generally believed to occur in MS patients when coordination of muscles by the nervous system is impaired and too many muscles, or the wrong muscles, contract involuntarily. In mild cases, spasticity may only be noticeable as a feeling of tightness, stiff muscles, pain or increased muscle tone, however, spasticity is often progressive and can build to locked limbs, contracted joints, painful spasms, or other uncontrollable muscle contractions over time.

Spasticity can generally interfere with any aspect of mobility including both involuntary and voluntary movements, and while it is uncertain how the symptoms of MS are related, it is believed that spasticity may contribute to or otherwise exacerbate other symptoms and complications. For example, spasticity, and related degeneration of nerves, may contribute to the onset and progression of symptoms like fatigue, muscle weakness, numbness, tingling, dizziness, pressure sores, aching joints and other pain by making normal movement difficult and reducing the thousands of small movements healthy individuals make both in sleep and while sitting down.

Nighttime spasticity also commonly contributes to sleep deprivation and/or poor sleep quality in MS patients, for example due to spasms waking the patient during the night or tense muscles preventing the patient from being comfortable enough to sleep, thereby intensifying or causing fatigue, pain, weakness, dizziness and/or depression.

The occurrence of spasticity itself may also be influenced by additional factors or triggers, such as sudden movements or position changes, temperature changes, infections, skin irritation, pain and stress. Unfortunately, the overlap of spasticity triggers and complications can set off a dangerous spiraling and/or escalation of symptoms.

As with MS overall, the primary goal of existing spasticity treatments is to reduce the negative effects as much as possible through physical therapy, medication, electrical stimulation therapies, or surgery. The use of physical therapy to ease spasticity generally focuses on exercises and movements that can increase flexibility, such as stretches and range of motion exercises, which may be impeded by the need to also take care to avoid spasticity triggers during movement. Unfortunately, physical therapy does not reduce spasticity in all MS patients and the exercise regimens can be difficult for some patients to maintain due to their physical state, requirements for additional equipment and supervision, and the possible triggering of an increase in symptoms.

The use of medication in treating spasticity often relies on suppressing or regulating nerve signals which, while temporary, replaces the effects of spasticity with other less severe side effects. For example, baclofen and tizanidine are two medications that relieve spasticity by attenuation of pathological overactive mono- and polysynaptic reflex arcs in the spinal cord or suppression of spinal and supraspinal reflexes by presynaptic inhibition. These effects may cause relaxation of muscles, both normal and spastic, commonly substituting general drowsiness and muscle weakness associated with the medications for the specific pain and contractions associated with spasticity.

The use of medication becomes particularly challenging as symptoms progress, as increased dosages required for achieving the same relief from spasticity cause increasingly greater side effects. As such, treatments using medications and chemical compositions must be based on balancing the positive and negative effects of the medication, which can be difficult to maintain due to the variation in intensity of spasticity from moment to moment and progression of the symptoms over time.

Electrical stimulation therapies and surgical interventions are specifically focused on the direct management and suppression of nerve fibers in MS patients. Electrical stimulation can be used on the nerves to affect the way signals are transmitted to the brain, potentially easing pain, but the positive effects are not well established and are limited to pain reduction without reducing the occurrence of spasticity. Surgical treatments require severing nerve roots or tendons for permanent relief of spasticity and result in severe and permanent side effects. Just as a patient will not suffer from spasticity in the muscles targeted with surgical intervention, the patient will lose substantial control or utility of the same muscle while neurodegeneration continues in the body.

While not identical, incidence of spasticity related to brain injury, spinal cord injury, cerebral palsy, stroke, fibromyalgia, myalgic encephalomyelitis/chronic fatigue syndrome, neuromyelitis optica, Parkinson's, amyotrophic lateral sclerosis, and other diseases or injuries of the nervous system is similarly difficult to treat or otherwise mitigate as that related to MS. Typical efforts to mitigate spasticity take on some combination of the treatments described above with the objective of reducing pain and discomfort associated with spasticity, but there is no recognized treatment that shows substantial reversal in the underlying spasticity itself. A treatment plan for a specific patient must also be closely monitored and adjusted to compensate for progression in the patient's spasticity and changes in side effects from the treatments.

There remains a need for a method of treating spasticity with reduced side effects. An ideal treatment would accomplish a reduction in not only the pain and difficulties associated with spasticity, but also in the frequency and severity of its occurrence and in the related impairment to motor control. Especially desired would be a treatment that may address underlying factors causing spasticity, such as nerve damage and/or loss of voluntary muscle control. By addressing the underlying cause of spasticity, rather than treating only the painful symptom, further improvements to mobility and overall quality of life may be achieved for patients that have previously had no hope for such improvements.

Existing treatments for spasticity and related neurodegenerative diseases would benefit from a method of treatment that reduces, stops, or reverses progression of degeneration in the nerves. There is a need for methods of treatment that can improve the efficacy of existing medications and physical therapies, such as by synergistically improving nervous function in the muscles and the spinal cord, in order to reduce dosage levels and related side effects.

Further, there is a need for a simple solution that can be implemented without increasing the level of intervention required by a caregiver or medical professional, while still being adaptable to the specific needs and symptoms of a given patient. Such a treatment or solution would clearly improve over the current state of the art treatments and solutions for spasticity, particularly in patients suffering from injuries or diseases affecting the central nervous system, including brain injury, spinal cord injury, cerebral palsy, stroke, fibromyalgia, myalgic encephalomyelitis/chronic fatigue syndrome, neuromyelitis optica, Parkinson's, amyotrophic lateral sclerosis, and MS.

### SUMMARY

The present disclosure comprises the discovery that a patient suffering from spasticity related to diseases of and injuries to the central nervous system can be treated using negative pressure therapy. It has surprisingly been found that when negative pressure therapy according to embodiments of the current disclosure is applied to a patient suffering from spasticity, the duration, intensity and frequency of spasticity can be significantly reduced. Embodiments of the present disclosure have further discovered new and unexpected improvements in spasticity and related symptoms from the combination of negative pressure therapy and existing medications in a treatment regime. The combination of negative pressure therapy with medication surprisingly reduces incidence of side effects from the medication and allows for a lower effect dose of medication. The improvements have surprisingly been shown across a range of conditions and medications.

An object of the disclosure is to treat spasticity and related conditions, including the involuntary and sustained contractions of muscles, such as associated with muscle stiffness, cramps, exaggerated movement and reflexes, clonus, or spasms, particularly in patients suffering from an injury or disease of the central nervous system. The disclosure also relates to methods of administering negative pressure therapy to treat spasticity.

The present disclosure describes an association between increased blood flow, increased oxygenation of tissue, and repeated change in sensory feedback resulting from negative pressure therapy and a reduction in intensity, duration, and frequency of spasticity.

According to the embodiments described herein, negative pressure therapy may be applied as a preventative treatment to prevent the occurrence of spasticity, as an immediate treatment of spasticity that has occurred or is occurring in a patient, or as part of a comprehensive and regular treatment plan for a patient having an overlying condition that can result in spasticity (e.g., MS, brain injury, spinal cord injury, cerebral palsy, stroke, fibromyalgia, myalgic encephalomyelitis/chronic fatigue syndrome, neuromyelitis optica, Parkinson's, amyotrophic lateral sclerosis, etc.).

WO 2019/064288 A1 discloses a medical device for pressure therapy.

CHANG ERIC ET AL: CRITICAL REVIEWS IN PHYSICAL AND REHABILITATION MEDICINE, vol. 25, no. 1-2, 1 January 2013 (2013-01-01), pages 11-22 is a review article on spasticity treatment.

SUNDBY ØYVIND HEIBERG ET AL,PLOS ONE, vol. 12, no. 6, 7 June 2017 (2017-06-07), pages 1-16 reports on acute effects of lower limb intermittent negative pressure on foot macro- and microcirculation in patients with peripheral arterial disease.

A first aspect of the invention provides an anti-spasmodic composition for use in a method of treating or preventing spasticity in a patient, the method comprising administering negative pressure therapy to the patient. The method may further comprise the use or administration of a pharmacological composition in combination with regular administration of negative pressure therapy. Such pharmacological compositions may include antispasmodic compositions or others known for neurological effects, including baclofen, tizanidine, gabapentin, diazepam, nabiximols, dantrolene, clonidine, cannabinoids (OCE, THC, Sativex, etc.), corticosteroids, cytoxan, imuran, ampyra, additional interferon beta medications, and other medications that would be understood by one skilled in the art from the present disclosure. Further pharmacological compositions may benefit from combination with negative pressure therapy in a treatment regime, although in differing ways, such as ocrelizumab, glatiramer acetate (Copaxone, Glatopa), or other immunosuppressive compositions.

In embodiments, a pressure therapy device may be arranged for applying pressure therapy to a user, particularly over a limb or area of a patient's body. The limb or area of the patient's body subjected to negative pressure may be selected as corresponding to or being near an occurrence of spasticity. The pressure therapy device may be configured to apply a negative pressure to the limb or the area of the patient's body for a predetermined amount of time and at a predetermined level of pressure.

According to the present disclosure, negative pressure therapy may be applied to an extremity of a limb where the spasticity has or is occurring, or to an extremity of a limb that is most closely associated with a region of spasticity. While embodiments are considered where negative pressure therapy is applied to only a single limb, the disclosure is not limited thereto and may include sequential and/or simultaneous application of pressure therapy to one or several regions of a patient's body.

Surprisingly, it has been discovered that application of negative pressure therapy according to embodiments of the current disclosure may provide an overall reduction in spasticity in the body, even where the negative pressure therapy is applied to only a certain limb or limbs. The systemic improvements in symptoms evidence a substantive improvement in neurological function and/or health beyond the limb, area or region of the body subjected to negative pressure therapy according to the current disclosure.

Negative pressure therapy according to the embodiments of the current disclosure may include application of a pulsating, or intermittent, pressure. The pulsating pressure may include generating a negative pressure for a first time interval and releasing the negative pressure for a second time interval. The release of the negative pressure during the second time interval may be configured to apply a different pressure from the negative pressure of the first time interval, such as a different negative pressure, an atmospheric pressure, an environmental pressure, or a positive pressure. The first time interval may be longer than a second time interval, may have the same length, or may be shorter.

In embodiments of the method, the duration of application of negative pressure therapy to the patient may be limited to the duration of symptoms or may be configured to a predetermined period of time according to the needs of the patient. The frequency of application of negative pressure may be limited to the frequency of symptoms or may be configured to a predetermined schedule according to the needs of the patient. In one example, negative pressure may be applied regularly prior to sleep, upon waking, and/or upon the occurrence of known spasticity triggers. Alternatively, negative pressure therapy according to the current disclosure may be applied on a regular scheduled basis, such as daily, or twice a day. The negative pressure therapy may be applied at the same time each day, or every other day, or every week as part of a regular schedule of treatment. It is noted that the application of negative pressure therapy on a regular basis does not exclude additional applications, such as on-demand or with a modification of the regular schedule of treatment over time, for example with decreasing frequency as symptoms improve.

The treatment of spasticity using negative pressure therapy according to the present disclosure has advantageously been found to provide a simple and adaptable method for substantially reducing or preventing spasticity according to the needs of an individual patient. Methods of the current disclosure have been found to quickly treat existing indications of spasticity while reducing future occurrences and their severity. Surprisingly, the benefits of a method including negative pressure therapy according to the current application have been shown to provide systemic improvements to the patient, not only for symptoms of spasticity but extending to additional symptoms of a neurodegenerative disease.

Methods of the current disclosure advantageously provide benefits to both affected and non-affected regions of the body without introducing any new adverse side effects while reducing the side effects related to concurrently applied pharmacological compositions or treatments, advantageously improving patient compliance and outcomes. While not completely understood, the treatment of spasticity using negative pressure therapy may also improve other symptoms associated with MS, related diseases and/or damaged central nervous systems, at least due to the interrelated progression of said symptoms.

Methods of the present disclosure may be applied concurrently or in succession with other known treatments or therapies for spasticity, or with known methods for reducing spasticity triggers. Advantageously, the coordinated application of negative pressure therapy with especially pharmacological compositions increases the effectiveness of the pharmacological compositions, such as by lowering an effective dose, while reducing the associated side effects thereof.

The negative pressure therapy according to the present disclosure may be coupled with a heating or cooling unit, vibration unit, orthotic device, brace, electrical stimulation unit, etc.

In another aspect, methods of the present disclosure may be applied with sensors arranged to monitor the effect of the treatment on the patient and/or to adapt the treatment to the patient. For example, blood flow sensors, temperature sensors, electrical sensors, etc. may be used to monitor the effect of the negative pressure therapy on the patient and to adjust the pressure, duration, or other parameters according to the patient's needs, such as through the use of a control module or other computing device.

According to varying embodiments, a control device may be provided for operating a pressure therapy device to perform the methods of the current application. The control device may be provided as a mobile device, such as a smartphone, laptop, tablet computer, or similar device. The control device may be arranged to control application of negative pressure in the pressure therapy device and/or to monitor and report use of the negative pressure device, such as to a clinician to ensure appropriate treatment or patient compliance.

These and other features, aspects, and advantages of the present disclosure will become better understood regarding the following description, appended claims, and accompanying drawings.

### BRIEF DESCRIPTION OF THE DRAWINGS

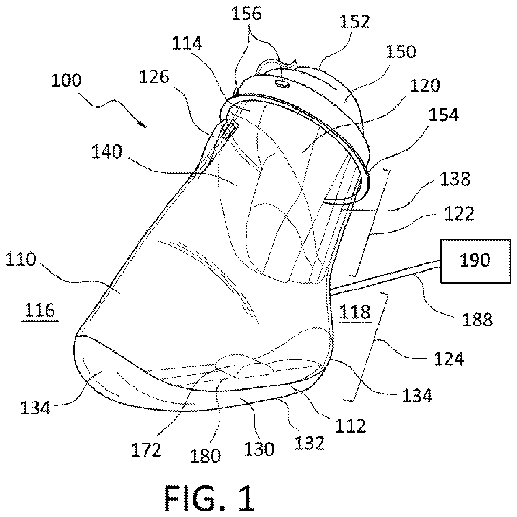

Fig. 1 shows a perspective view of a pressure therapy device.

### DETAILED DESCRIPTION OF VARIOUS EMBODIMENTS

While the disclosure is susceptible to various modifications and alternative constructions, certain illustrative embodiments are described below. It should be understood, however, there is no intention to limit the disclosure to the embodiments disclosed, but on the contrary, the intention is to cover all modifications, alternative constructions, combinations, and equivalents falling within the spirit and scope of the disclosure and defined by the appended claims.

It will be understood that, unless a term is defined in this patent to possess a described meaning, there is no intent to limit the meaning of such term, either expressly or indirectly, beyond its plain or ordinary meaning.

Although exemplary embodiments of the disclosure are described for treating a patient experiencing spasticity as a result of MS, the embodiments of the disclosure may also be adapted to treat spasticity resulting from different types and severities of disease or trauma of the central nervous system as discussed in the background of the application. In like manner, the embodiments of the disclosure may be adapted for treating additional symptoms of MS and related diseases or injuries, such as pain, limited flexibility, fatigue, depression, loss of nerve control or feeling in muscles and limbs, whether or not said further symptoms are associated with spasticity. Method steps for treating spasticity using negative pressure therapy may be configured as stand-alone steps for treatment or may be readily adapted to be incorporated with another treatment or therapy.

An "anti-spasmodic composition" according to the present disclosure may comprise any known pharmacological composition used for the treatment of spasticity in injuries or diseases affecting the central nervous system, including brain injury, spinal cord injury, cerebral palsy, stroke, fibromyalgia, myalgic encephalomyelitis/chronic fatigue syndrome, neuromyelitis optica, Parkinson's, amyotrophic lateral sclerosis, multiple sclerosis (MS) and additional neurodegenerative diseases. For example, an anti-spasmodic composition may comprise baclofen, tizanidine, gabapentin, diazepam, nabiximols, dantrolene, clonidine, cannabinoids (OCE, THC, Sativex, etc.), corticosteroids, cytoxan, imuran, ampyra, dimethyl fumarate (Tecfidera), diroximel fumarate (Vumerity), phenibut, additional interferon beta medications, and similar pharmacological compositions, whether administered as an injectable treatment, an oral treatment or an infusion treatment. Dosage of the anti-spasmodic compositions may be according to the level of one skilled in the art, but may further be reduced in view of the advantages of the current disclosure.

Further pharmacological compositions are contemplated as "immunosuppressive compositions," that act to suppress autoimmune attacks on the nervous system and may benefit from combination with negative pressure therapy in a treatment regime, although in differing ways than with anti-spasmodic compositions. Immunosuppressive compositions may comprise any known pharmacological composition used for the treatment of or prevention of relapse in injuries and diseases affecting the central nervous system, including brain injury, spinal cord injury, cerebral palsy, stroke, fibromyalgia, myalgic encephalomyelitis/chronic fatigue syndrome, neuromyelitis optica, Parkinson's, amyotrophic lateral sclerosis, multiple sclerosis (MS) and additional neurodegenerative diseases. For example, an immunosuppressive compositions may comprise ocrelizumab, glatiramer acetate (Copaxone, Glatopa), fingolimod (Gilenya), teriflunomide (Aubagio), siponimod (Mayzent), cladribine (Mavenclad), natalizumab (Tysabri), alemtuzumab (Campath, Lemtrada) or other immunosuppressive pharmacological compositions, whether administered as an injectable treatment, an oral treatment or an infusion treatment. Dosage of the immunosuppressive compositions may be according to the level of one skilled in the art, but may further be reduced in view of the advantages of the current disclosure.

The term "morning" is used herein to describe a time early in the day after the patient has awakened from overnight sleep, generally between about 6:00 am and 11:00 am. In one embodiment, negative pressure therapy may be administered at 8:30 am, before or after rising from overnight sleep. The term "evening" is used herein to describe a time later in the day before the patient goes to bed for sleep at night, generally between about 7:00 pm and 10:00 pm. In one embodiment, negative pressure therapy may be administered at 7:00 pm, before or after the patient lays down to sleep.

Reference to "a limb" as used in the present disclosure can be any part of a human or animal body that can be easily introduced into a pressure therapy device as described in the disclosure. A limb can comprise an arm or leg, a portion of an arm or leg (e.g. a forearm, hand, lower leg, or foot), or more than one of such parts of the body. While the pressure therapy device is described within the context of an embodiment directed to a lower leg and foot, many features described herein may be extended to pressure therapy devices and components that secure other limbs and body parts, for performing methods of the disclosure.

For explanatory purposes, embodiments of the current disclosure may refer to general anatomical terms for the human body. Such anatomical terms are provided to distinguish regions of the body from one another but are not to be considered to limit the scope of the disclosure. The terms "proximal" and "distal" generally refer to locations that correspond to the location on a limb relative to the point of attachment of the limb to the body. The terms "upper" and "lower" may be used in combination with "proximal" and "distal" to connote gradations in location of "proximal" and "distal."

Embodiments of a method of treating or preventing spasticity in a patient are provided for reducing the impact or reversing the occurrence of spasticity in the patient's muscles, the method comprising administering negative pressure therapy to the patient. Additional embodiments may comprise the use of an anti-spasmodic composition for treating spasticity and related symptoms in a patient, where negative pressure therapy is administered to the patient to improve the effects of the anti-spasmodic composition.

The embodiments of the method advantageously allow for treatment of spasticity with a reduced need for drugs or invasive surgery and a reduced occurrence of related side effects. According to varying embodiments, some patients may even be treated for spasticity without the use of drugs or surgery at all. The methods further treat spasticity through interaction with both affected tissue and a corresponding portion of the nervous system, enabling a treatment of the underlying cause and related effects of spasticity rather than only reducing pain. As such, the disclosed methods provide the unexpected results of improved walking abilities, improved stability, reduced pain, improved sleep, improved mood, reduced fatigue, and additional systemic improvements.

Further benefits of the described embodiments may include improved peripheral blood flow, healthier arteries, improved endothelial function, decreased lactate, improved mitochondrial activity, and reduction or prevention of chronic inflammation.

MS and related injuries and diseases of the central nervous system causing spasticity are generally believed to damage the ability of the central nervous system to regulate nerve signals, resulting in involuntary and painful contraction or release of muscles. Among the considered causes of spasticity, it is believed that local conditions in a region of a patient's body may deteriorate, resulting in undesirable ion channel openings as well as disturbed transmitter break-down, release or uptake. Local conditions can impact sensory endings leading or contributing to inaccurate sensory feedback, hypersensitivity, or dysfunctional signals causing spasticity.

Without being bound by any theory, the unexpected and surprising effects of treating spasticity with negative pressure therapy is believed to arise in part as a consequence of a generation of repeated changes in sensory feedback from the peripheral nervous system of a patient. This sensory input is believed to work on neural networks of the spinal cord to influence hyperexcitability of interneurons, motor neurons or other elements of the central nervous system that contribute to the occurrence of spasticity.

Notably, vascular nerves in the veins of the body are activated to a greater degree in vasodilation, meaning that the increased blood flow caused by a negative pressure environment has an outsized, nearly immediate impact on sensory input received by the central nervous system. The combined effect of stimulating vascular nerves, sensory nerves of the skin, and nerves of the muscles caused by negative pressure therapy can extend simultaneously over a large portion of the patient's body and/or vascular system, such that a strong signal or sensory input is provided over and within large parts of the body at regular, or intermittent, intervals.

It is believed that the strong, intermittent sensory input caused by negative pressure therapy can interrupt dysfunctional or incomplete nerve signals commonly associated with spasticity and may unexpectedly increase the ability of the central nervous system to regulate nerve signals with repeated use. In this manner, sensory input provided by the negative pressure therapy may surprisingly serve to essentially "reset" portions of the nervous system, particularly those associated with spasticity, and/or to in effect "exercise" these portions of the nervous system to reduce occurrence of undesirable ion channel openings as well as disturbed transmitter break-down, release or uptake, and improving sensory feedback, sensitivity, and signal function of the nerves.

The described advantageous effects on the nervous system may be coordinated with administration of anti-spasmodic compositions, to achieve a surprising combined or synergistic impact on spasticity as well as related symptoms and conditions. While not bound by any particular theory, anti-spasmodic compositions may function by activating or agonizing receptors in the nervous system to decrease the frequency and/or scale of spasticity, and the concurrent improvements in sensory input and processing related to negative pressure therapy of the present disclosure may surprisingly strengthen the activating or agonizing effect on the receptors. Increased, result effective uptake of the anti-spasmodic compositions may be a product of increased health or coordination in the receptors, such that a result effective dosage, and corresponding side effects, may be reduced as a result of the combined application of anti-spasmodic compositions and negative pressure therapy. A summary of some anti-spasmodic compositions and their related effects is provided in the Table I below (see also Patejdl Robert, Zettl Uwe K., Spasticity in multiple sclerosis: Contribution of inflammation, autoimmune mediated neuronal damage and therapeutic interventions, Autoimmunity Reviews (2017))*.*

**Table I**

| Active Substance | Receptor Binding | Mechanism of Action | Advantages | Disadvantages |
|---|---|---|---|---|
| Baclofen | Agonist of cerebral and spinal GABA-b receptors | Attenuation of pathological overactive mono-and polysynaptic reflex arcs in the spinal cord | Decreases frequency of muscle spasms and spasticity induced pain, reduces clonus, increases joint mobility; intrathecal application with less systemic effects is also effective and safe | Deterioration of postural stability; individual needed dose highly variable, limited by side effects such as fatigue and dizziness |
| Tizanidine | α₂-receptor agonist with main point of action in the spinal cord | Suppression of spinal and supraspinal reflexes by presynaptic inhibition | Less negative influence on the muscle power than baclofen | risk of hepatic dysfunction; hypotonia; simultaneous treatment with SSRI contraindicated |
| Gabapentin | Central I-type calcium channels | Inhibition of excitatory transmitter release and neuronal excitability | Few serious side effects | Drowsiness, dizziness |
| Nabiximols | Agonists on CB₂-receptors presynaptic membranes of central neurons | Inhibition of excitatory transmitter release, reduction of stretch reflex responses and corticospinal excitability | Admitted as add-on therapy, good antispasmodic effect in 50% of patients | Dizziness and fatigue; limitation of roadworthiness and travel capability |
| Dantrolene | Ryanodine receptor antagonists | Reduced contractility, inhibition of excitatory transmitter release | Alternative drugs at the time, useful when effects of other substances are insufficient, contraindications exist or intolerable side effects of the above mentioned oral antispasmodics occur | Pronounced muscle weakness (dantrolene), dizziness, orthostatic hypothia (clonidine) |
| Clonidine | α₂-receptor agonists | | | |

Use of an anti-spasmodic composition for treating spasticity resulting from MS or related conditions according to the present disclosure may preferably include the concurrent application of negative pressure therapy. For example, an anti-spasmodic composition may be administered to a patient on a daily basis according to a result-effective dosage and schedule, such that the combination of negative pressure therapy to the patient with the anti-spasmodic composition may allow for administration of a reduced dosage for achieving the same or greater benefits while reducing or preventing unwanted side effects. In another example, negative pressure therapy may be administered to a patient according to a regular schedule, and an anti-spasmodic composition may be administered to the patient by individual titration, starting at a low dosage and increasing gradually until an optimum effect is achieved.

Notably, the use of an anti-spasmodic composition may reduce the dosage needed for administration to a patient, whether the dosage is reduced from a previously administered level or reduced relative to an expected level. The use of an anti-spasmodic composition for treatment of MS or related conditions using application of negative pressure therapy may further demonstrate synergistic effects with the anti-spasmodic composition, such that the further improvements in symptoms or even reversal of symptom progression may result.

In a similar manner, one skilled in the art considering the above benefits of the combined application of anti-spasmodic compositions and the negative pressure therapy would expect favorable results from combining immunosuppressive compositions with negative pressure therapy, with or without anti-spasmodic compositions as well. Immunosuppressive compositions used in treating MS and related conditions serve to inhibit autoimmune attacks on the nervous system, while the concurrent application of negative pressure therapy according to the methods of the current disclosure may serve to repair or inhibit damage to the nervous system according to the benefits described above.

Of particular interest in the use of negative pressure therapy for the treatment of spasticity related to MS and similar conditions is the impact of increased sensory input on neural networks that produce rhythmic activation of muscles to control the limbs, commonly referred to as central pattern generators (CPGs). These generators are generally believed to be influenced by various factors including sensory input from the peripheral nervous system.

In patients having MS and related conditions, it is believed that inhibitory signals (from inhibitor interneurons) that normally restrict or limit undesired motor neuron activity are reduced due to damage to the central nervous system, allowing increased motor neuron activity to activate CPGs and lead to spasticity. The strong sensory input resulting from the administration of negative pressure therapy is believed to synchronize and/or strengthen the inhibitory effects from inhibitor interneurons such that over-activation or hyper-sensitivity of background motor neurons is prevented. Such a synchronization and/or strengthening of the inhibitory effects from inhibitor interneurons may be particularly beneficial in patient populations relying on anti-spasmodic compositions for treatment. As discussed above, the surprising improvement of synchronization and/or strengthening of the inhibitory effects from inhibitor interneurons resulting from negative pressure therapy may increase the corresponding inhibitory or neurological effects of anti-spasmodic compositions, leading to a faster, more effective response to the anti-spasmodic compositions in treatment of spasticity and related conditions.

Further benefits to patient's suffering from spasticity are believed to include an overall increase in the condition of peripheral tissue, leading to healthier function. In particular, increased oxygenation of tissue and blood flow may allow peripheral tissue to better regulate and control released transmitters that could otherwise cause contractions associated with spasticity. Additional benefits corresponding to links between blood flow and spasticity, and peripheral sensory input and spasticity, are contemplated and may be realized by the treatment of spasticity using negative pressure therapy according to methods of the current disclosure.

Negative pressure therapy according to the embodiments of the current disclosure may include application of a pulsating pressure or repeated, alternating introduction of two or more different pressures during consecutive time periods. The pulsating pressure may include generating a negative pressure for a first time interval and releasing the negative pressure for a second time interval. The release of the negative pressure during the second time interval may be configured to apply a different pressure from the negative pressure of the first time interval, such as a different negative pressure, an atmospheric pressure, an environmental pressure, or a positive pressure. The first time interval may be longer than a second time interval, may have the same length, or may be shorter.

In one example, a pulsating pressure can comprise the alternating introduction of an applied pressure and release of the applied pressure to return to approximately atmospheric pressure. The applied pressure can be a positive pressure or a negative pressure. In embodiments using a negative pressure, the period during which the negative pressure is introduced and is present is the negative pressure period. Likewise, in systems utilizing a positive pressure, the period during which the positive pressure is introduced and is present is the positive pressure period. In each case, the period during which the applied pressure is released, and atmospheric pressure is returned and is present is the atmospheric pressure period.

Embodiments discussed herein are discussed referring to a negative applied pressure. The disclosure should not be construed to exclude devices and methods using a positive pressure rather than a negative pressure. Further, the disclosed embodiments are not restricted to the use of pressure, but may include alternative methods for activating a vasodilation reflex in the body in an intermittent manner.

In some embodiments, multiple, consecutive, alternating negative pressure periods and atmospheric pressure periods are applied to a limb within a pressure chamber without removing the limb from the chamber. The negative and atmospheric pressure periods can be of the same or a different duration. In some embodiments, the negative pressure periods and atmospheric pressure periods can be selected according to known methods, such as those described in commonly owned U.S. Patent No. 7,833,179, issued November 16, 2010.

In some embodiments the negative pressure period is between 1 second and 20 seconds in duration and the atmospheric pressure period is between 2 seconds and 15 seconds in duration. Further, in some embodiments, the negative pressure period is between 5 seconds and 15 seconds in duration and the atmospheric pressure period is between 5 seconds and 10 seconds in duration. And in some preferred embodiments, the negative pressure period is approximately 10 seconds in duration and the atmospheric pressure period is approximately 7 seconds in duration.

The pressure applied within the pressure chamber can be fixed or selected at the point of use. Embodiments of devices and methods according to the present disclosure provide for applying a negative pressure of -150 mmHg or less, more particularly -80 mmHg (-10.7 kPa) or less. In some embodiments, the negative pressure can be -60 mmHg (-8.0 kPa) or less. Some embodiments utilize a negative pressure of approximately -40 mmHg (-5.3 kPa).

The preferred negative pressures have been selected to reduce complications that might arise from applying higher negative pressures. In some embodiments, a negative pressure has been selected to encourage local vasodilation in the surface of the limb while minimizing the risk of possible complications. Pulsating the negative pressure has been found to encourage blood flow and a pulsating negative pressure of 0 to -40 mmHg (0 to -5.3 kPa) is preferably generated in the pressure chamber.

In an embodiment, negative pressure therapy may include arranging a pressure therapy device for applying negative pressure therapy to a region of a patient's body, for example a limb of patient, such as a lower leg or an arm. The region of the patient's body may be selected for receiving negative pressure therapy due to the occurrence of spasticity in that region or due to proximity to a region of the patient's body experiencing spasticity. In one example, a patient's lower leg may be subjected to negative pressure therapy in order to treat spasticity occurring in an upper leg or lower back of the patient.

According to embodiments, the pressure therapy device may comprise any suitable device for applying negative pressure to a region of a patient's body. Fig. 1 illustrates one example of a pressure therapy device 100 from co-owned U.S. Patent No. 10,940,075, issued March 9, 2021.

As shown in Fig. 1, the pressure therapy device may include a pressure chamber 110. The pressure chamber 110 may be arranged for enclosing the patient's limb between first and second ends 112, 114 and anterior and posterior surfaces 116, 118 of the pressure chamber 110. In the embodiment of Fig. 1, the second end 114 defines an opening 120 to an interior region 122, 124 inside the pressure chamber 110.

The interior region 124 of the pressure chamber 110 may communicate with a pump unit 190 through a conduit 188 at a side 116, 118 of the pressure chamber 110, the pump unit 190 being configured for increasing and decreasing a pressure of the interior region 122, 124 of the pressure chamber 110. An inflatable padding 140 may secure to the opening 120 of the pressure chamber 110 to grip the limb of the patient while a seal 150 secures to the pressure chamber 110, surrounding the opening 120 and extending beyond the pressure chamber 110 in a proximal direction, such that the seal 150 affixes firmly about the limb of the patient and closes the pressure chamber 110 from ambient pressure.

As depicted in Figs. 1, the pressure chamber 110 may comprise a support surface 130 at the second end 114. The support surface 130 may comprise a flat bottom portion 132 and angled portions 134 and may be molded as a separate part fixed to the pressure chamber 110. The flat bottom portion 132 allows the pressure chamber 110 to remain stable in a predetermined position such that a user may be seated comfortably during use of the pressure therapy device 100. The angled portions 134 may be present at the anterior and posterior surfaces 116, 118 such that the pressure chamber 110 can be stably positioned in a tilted position. The angled portions 134 may be curved, such that the pressure chamber 110 may be easily rotated, or may be flat to provide stability in a predetermined position. The limb may then contact the positioning mechanism 172 within the pressure chamber 110 and the pressure chamber 110 may rotate to lie on the flat portion of the lower surface 130.

The support surface 130 may comprise a modular space 180 for receiving one or more modular components. In one embodiment the modular components may be interchangeable and may include a vibration component, heating component, cooling component, etc.

The seal 150 may have a frustoconical shape including a proximal end 152 and a distal end 154. The proximal end 152 may have a center axis that is eccentric to a center axis of the distal end 154, such that a limb is positioned more in a posterior portion of the opening 120. When secured about the limb of a user, a pull tab 157 may be configured to extend along the distal length of the seal 150 to enable a user to grip and open the seal 150 about the limb. The proximal end 152 of the seal 150 may be rolled back in a distal direction to widen an opening of the seal 150 for placement or removal of a limb. Protrusions 156 may be positioned on the distal end 154 to frictionally retain the seal 150 in a rolled position such that a user does not have to hold the seal 150 back during placement of a limb. This feature enables the use of the pressure therapy device 100 by individuals that do not have sufficient strength, dexterity, or mobility to manipulate a seal while also maneuvering a limb or the pressure chamber 110.

According to varying embodiments, the pump unit 190 may include or be operatively connected with sensors arranged to monitor the effect of the treatment on the patient and/or to adapt the treatment to the patient. For example, blood flow sensors, temperature sensors, electrical sensors, etc. may be used to monitor the effect of the negative pressure therapy on the patient and to adjust the pressure, duration, or other parameters according to the patient's needs, such as through the use of a control module or other computing device associated with the pump unit 190. A control device of the pump unit 190 may be provided for operating a pressure therapy device to perform the methods of the current application. The control device may be provided as a mobile device, such as a smartphone, laptop, tablet computer, or similar device within or operatively connected with the pump unit 190. The control device may be arranged to control application of negative pressure in the pressure therapy device and/or to monitor and report use of the negative pressure device, such as to a clinician to ensure appropriate treatment or patient compliance.

In an embodiment, treatment of spasticity using negative pressure therapy may begin at the first sign of spasticity. Signs of spasticity may include increased muscle tone, cramps, spasms or other involuntary muscle contractions, but can further include tingling sensations or other feelings identified by a patient as preceding or accompanying spasticity in their muscles.

The duration of negative pressure therapy applied to the patient may correspond to the spasticity experienced by the patient, such that application of negative pressure therapy continues until symptoms have completely subsided. Alternatively, a set time period beyond the cessation of symptoms may be selected, for example five minutes, ten minutes or twenty minutes after the cessation of symptoms.

In another aspect of the method, negative pressure therapy may be applied as a preventative measure to prevent the occurrence of spasticity symptoms. In one example, a patient may undergo treatment using negative pressure therapy prior to known spasticity triggers, including change in temperature or posture, or in response to spasticity triggers that cannot be otherwise controlled, such as infection or pain. In one aspect, a regular treatment of the patient using negative pressure therapy is contemplated where the patient uses negative pressure therapy for a regular period at least once per day.

Embodiments may also include the use of an anti-spasmodic composition for the treatment of spasticity or related conditions, as described herein. The method may include administering the anti-spasmodic composition and applying negative pressure therapy to increase the efficacy of the anti-spasmodic composition. In one example, a patient may undergo treatment including a daily administration of the anti-spasmodic composition and a daily application of negative pressure therapy according to the instant application. A dosage amount of the anti-spasmodic composition may be reduced due to the combined or synergistic effects of the composition with the negative pressure therapy. In one aspect, the anti-spasmodic composition may be administered on-demand, such that negative pressure therapy may be applied corresponding to administration of the anti-spasmodic composition, such as at the same time or in the same day.

In varying embodiments, the negative pressure therapy may be applied to the patient within 24 hours of administration of the anti-spasmodic composition to the patient, more particularly within 18 hours, within 12 hours, within 8 hours, within 6 hours, within 4 hours, within 3 hours, within 1 hour or at the time of administration of the anti-spasmodic composition. Administration of the anti-spasmodic composition may be performed on a regular schedule, such as with a particular meal, each morning, or each night. The application of the negative pressure may be performed at the same time as the administration of the anti-spasmodic composition or on a related schedule, such as administration of the anti-spasmodic composition upon waking and application of negative pressure therapy upon lying down to sleep, or the like.

Initial administration of the anti-spasmodic composition may be performed at a result effective dose and in a result effective manner as would be understood by one skilled in the art considering the instant application, while subsequent administration may be performed at a lower dose. For example, an initial dose may be administered for a period of less than 6 months, less than 3 months, less than 1 month or less than two weeks with concurrent application of negative pressure therapy. As such, a subsequent lower dose of the anti-spasmodic composition may be applied after 6 months, after 3 months, after 1 month or after than two weeks of concurrent application of negative pressure therapy and the initial dose.

In one aspect, negative pressure therapy may be administered to a patient prior to sleep in order to prevent the occurrence of spasticity from disrupting sleep. In another aspect, the negative pressure therapy may be administered to a patient prior to rising from sleep, in order to prevent the occurrence of spasticity relating to the initial movement and weight bearing of the patient's muscles.

Preventative spasticity treatments using negative pressure therapy may be applied to at least one region of the patient's body for at least 20 minutes, for at least 30 minutes, for at least 40 minutes, or for at least one hour. According to some embodiments, preventative treatment may include one treatment for at least one region of the patient's body per day, two treatments per day, or at least two treatments per day. While embodiments are considered where negative pressure therapy is applied to only a single limb, the disclosure is not limited thereto and may include sequential and/or simultaneous application of pressure therapy to one or several regions of a patient's body.

The disclosed preventative treatments using negative pressure therapy may be provided as a regular and continuous part of a treatment regime for the duration of a disease or injury of the central nervous system, such as MS. Notably, while it is possible that the frequency and duration of the treatments may be reduced in some patients without a corresponding increase in spasticity or related symptoms, it is preferred that the treatments be applied in a regular and continuous manner to achieve the greatest level of prevention and recovery.

Methods of the current disclosure advantageously provide benefits to both affected and non-affected regions of the body without any adverse side effects, improving patient compliance and outcomes. While not completely understood, the treatment of spasticity using negative pressure therapy may also improve other symptoms associated with MS or damaged central nervous systems, at least due to the interrelated progression of said symptoms. Similarly, the treatment of spasticity using anti-spasmodic compositions administered with negative pressure therapy may also improve other symptoms associated with MS or damaged central nervous systems, at least due to the interrelated effects and benefits of the anti-spasmodic compositions and negative pressure therapy.

As would be understood by one of ordinary skill in the art from the present disclosure, the described methods of treating spasticity using negative pressure therapy may be applied concurrently or in succession with other known treatments or therapies for spasticity, or with known methods for reducing spasticity triggers. For example, the negative pressure therapy according to the present disclosure may be coupled with a heating or cooling unit, vibration unit, orthotic device, brace, electrical stimulation unit, etc.

In another aspect, methods of the present disclosure may be applied with sensors arranged to monitor the effect of the treatment on the patient and/or to adapt the treatment to the patient. For example, blood flow sensors, temperature sensors, electrical sensors, etc. may be used to monitor the effect of the negative pressure therapy on the patient and to adjust the pressure, duration, or other parameters according to the patient's needs, such as through the use of a control module or other computing device.

A control device may be provided for operating a pressure therapy device to perform the methods of the current application. The control device may be provided as a mobile device, such as a smartphone, laptop, tablet computer, or similar device. The control device may be arranged to control application of negative pressure in the pressure therapy device and/or to monitor and report use of the negative pressure device, such as to a clinician to ensure appropriate treatment or patient compliance.

In an embodiment, the control device may include a challenge and response module configured to issue a challenge or query to the patient before, during or after operation of the pressure therapy device. The challenge or query may include information regarding a patient's symptoms or treatment parameters, and the patient's response may be provided to a remote server, such as for further processing or compilation. According to varying embodiments, a response to the challenge may be required for operation of the pressure therapy device. Further processing or compilation of the responses at the remote server may be used to determine operating parameters for use by the control device in operating the pressure therapy device, or for providing a recommended treatment or schedule to the patient.

Case studies have been employed to demonstrate the efficacy of negative pressure therapy for treating spasticity, including the use of anti-spasmodic compositions in combination with negative pressure therapy, according to the current disclosure and have yielded positive initial outcomes.

In one such study, a patient suffering from MS related spasticity in the form of overnight muscle cramps was administered negative pressure therapy in the form of pulsating negative pressure. The patient exhibited a history of severe spasticity related cramps in her legs. Generally, the cramps begin slowly and increase in severity as they extend from extremities up the leg over a period of 5-10 minutes. Both legs were commonly affected, whether at the same time or separately. Adding to the impact of her spasticity symptoms, the patient suffered attacks most frequently at night and struggled with an associated poor quality of sleep.

Prior to treatment using negative pressure therapy the patient relied on the use of muscle relaxing medications, or anti-spasmodic compositions, which generally required at least 20 minutes for pain relief and did not result in a reduction in the frequency of the cramps from one day to the next. While not as severe as the cramps, the medications exhibited common side-effects that would preferably be avoided.

Over an initial period of 25 days, negative pressure therapy according to the current disclosure was administered as a preventative treatment for spasticity. The treatment consisted of applying a pulsating negative pressure to one or both limbs for a period of one hour, forty minutes, or thirty minutes. The patient recorded the treatment administered and categorized any spasticity related cramps as mild, moderate or severe. Throughout the period of treatment, no cramping or other spasticity related effects were felt in a treated leg during the night, comprising a period of approximately eight hours. The use of muscle relaxing medications, or anti-spasmodic compositions, was reduced in frequency and dose without a decrease in positive effect, and while showing a reduction in observable side effects.

In contrast, when no negative pressure therapy was administered, either at all or to only one leg, cramping commonly occurred and increased in severity dependent on the length of time passed since treatment using negative pressure therapy. Accordingly, the use of muscle relaxing medications, or anti-spasmodic compositions, was similarly negatively affected, requiring increasing lengths of time for pain relief dependent on the length of time passed since treatment using negative pressure therapy.

The recorded treatment regimen and resulting categorization of spasticity cramps is shown in Table II below.

| **Table II - Negative Pressure Therapy (pre-bedtime use for Multiple Sclerosis related spasticity)** | | | | |
|---|---|---|---|---|
| **Day** | **Used?** | **Which Leg(s)?** | **Duration** | **Overnight Cramping / comments** |
| **1** | Y | L + R | 1 hr each | No spasticity |
| **2** | N | | | Severe spasticity cramps |
| **3** | Y | L+R | 1 hr each | No spasticity |
| **4** | Y | L+R | 1/2 hr L/1 hr R | No spasticity |
| **5** | Y | L | 1/2 hr | No spasticity in L (treated) Mild spasticity cramps in R (untreated) |
| **6** | Y | L+R | 1 hr each | No spasticity |
| **7** | Y | L | 1 hr L | No spasticity in L (treated) Mild spasticity cramps in R (untreated) |
| **8** | Y | | | No spasticity |
| **9** | N | | | Moderate spasticity cramps in both legs |
| **10** | Y | L+R | 1/2 hr each | No spasticity |
| **11** | N | | | No spasticity |
| **12** | Y | L+R | 40 min each | No spasticity |
| **13** | Y | L | 40 min | No spasticity |
| **14** | Y | L | 40 min | No spasticity in L (treated) Mild spasticity cramps in R (untreated) |
| **15** | Y | L | 40 min | No spasticity in L (treated) Moderate spasticity cramps in R (untreated) |
| **16** | Y | L | 40 min | No spasticity in L (treated) Moderate spasticity cramps in R (untreated) |
| **17** | Y | L | 40 min | No spasticity |
| **18** | Y | L | 40 min | No spasticity |
| **19** | N | | | No spasticity |
| **20** | N | | | Moderate spasticity cramps in both legs |
| **21** | Y | L+R | 1 hr each | No spasticity |
| **22** | Y | L | 40 min | No spasticity |
| **23** | Y | L | 40 min | No spasticity in L (treated) Mild spasticity cramps in R (untreated) |
| **24** | Y | L+R | 40 min each | No spasticity |
| **25** | Y | L | 40 min | No spasticity in L (treated) Mild spasticity cramps in R (untreated) |
| **26** | N | | | Moderate spasticity cramps in both legs (R worse than L) |
| **27** | N | | | No spasticity |
| **28** | N | | | Mild spasticity cramps in both legs |
| **29** | N | | | Moderate spasticity cramps in both legs |
| **30** | N | | | Moderate spasticity cramps in both legs |
| **31** | N | | | Moderate spasticity cramps in both legs |
| **32** | N | | | Moderate spasticity cramps in both legs |
| **33** | N | | | Severe spasticity cramps in both legs |
| **34** | N | | | Severe spasticity cramps in both legs |
| **35** | N | | | Severe spasticity cramps in both legs |

The patient further reported that administration of negative pressure therapy to existing spasticity cramping was effective in relieving the cramp in less than 5 minutes, such as during daytime use.

In another study, the effect of varying pressure levels on the limb of the user was analyzed by employing the pressure therapy device of the current disclosure to treat a sample population of 16 individuals suffering from peripheral arterial disease. Over 90 percent of the sample population in the study suffered from mild claudication and were categorized as stage II of the fontaine stages for chronic limb ischemia.

Pairwise comparisons of the impact of pressure levels -10 mmHg, -20 mmHg, -40 mmHg and -60 mmHg on flow and laser Doppler flux measurements of the leg and foot were performed, and demonstrated a statistically significant increase in flow and Doppler flux, as shown in Table III below.

**Table III**

| | Flow | | | | | Laser Doppler Flux | | | | |
|---|---|---|---|---|---|---|---|---|---|---|
| Sample 1 vs Sample 2 * | Test Statistic | Std. Error | Std. Test Statistic | p-value | Adjusted p-value # | Test Statistic | Std. Error | Std. Test Statistic | p-value | Adjusted p-value # |
| 0 vs -10 mmHg | -0.38 | 0.56 | -0.67 | 0.50 | 1.00 | -0.56 | 0.56 | -1.01 | 0.31 | 1.00 |
| 0 vs -20 mmHg | -1.31 | 0.56 | -2.35 | 0.02 | 0.19 | -1.06 | 0.56 | -1.90 | 0.06 | 0.57 |
| 0 vs -40 mmHg | -2.56 | 0.56 | -4.58 | <0.01 | <0.01 | -2.25 | 0.56 | -4.03 | <0.01 | <0.01 |
| 0 vs -60 mmHg | -3.25 | 0.56 | -5.81 | <0.01 | <0.01 | -2.38 | 0.56 | -4.25 | <0.01 | <0.01 |
| -10 mmHg vs -20 mmHg | -0.94 | 0.56 | -1.67 | 0.09 | 0.94 | -0.50 | 0.56 | -0.89 | 0.37 | 1.00 |
| -10 mmHg vs -40 mmHg | -2.19 | 0.56 | -3.91 | <0.01 | <0.01 | -1.69 | 0.56 | -3.02 | <0.01 | 0.03 |
| -10 mmHg vs -60 mmHg | -2.88 | 0.56 | -5.14 | <0.01 | <0.01 | -1,81 | 0.56 | -3.24 | 0.01 | 0.01 |
| -20 mmHg vs -40 mmHg | -1.25 | 0.56 | -2.24 | 0.03 | 0.25 | -1.19 | 0.56 | -2.12 | 0.03 | 0.34 |
| -20 mmHg vs -60 mmHg | -1.94 | 0.56 | -3.47 | <0.01 | <0.01 | -1.31 | 0.56 | -2.35 | 0.02 | 0.19 |
| -40 mmHg vs -60 mmHg | -0.69 | 0.56 | -1.23 | 0.22 | 1.00 | -0.13 | 0.56 | -0.22 | 0.82 | 1.00 |

As demonstrated, embodiments of the pressure therapy device utilizing a negative pressure of approximately -40 mmHg (-5.3 kPa) shows a significant improvement in blood flow through the limb of patients suffering from peripheral arterial disease.

As indicated in the current disclosure, it has now been surprisingly found that the increased blood flow and oxygenation may be additionally effective in alleviating and treating spasticity in patients suffering from spasticity related to injury or disease of the central nervous system. The use of negative pressure therapy according to the disclosed embodiments does not simply provide relief from pain or increase muscle relaxation but has surprisingly shown the new and unexpected result of improving nerve control in patients suffering from MS or related diseases and injuries affecting the central nervous system. No similar benefit has been achieved or otherwise contemplated prior to the discovery disclosed in the current application.

In a further observational study, negative pressure therapy according to methods of the current disclosure was applied to seven patients diagnosed with MS. Out of the seven patients, three were specifically diagnosed with secondary progressive MS (SPMS), two were specifically diagnosed with remitting-relapsing MS (RRMS), one was specifically diagnosed with primary progressive MS (PPMS), and one was not specifically diagnosed with a particular classification. Each of the patients was previously treating their condition with anti-spasmodic compositions, and use of the anti-spasmodic compositions continued during the study, although a reduction in dosage was observed in some patients.

Application of negative pressure therapy according to methods of the current disclosure results in favorable results. Six out of seven patients reported existing difficulties with spasticity and four out of the six patients (67%) reported improvements in at least the occurrence of stiffness and/or spasms in the legs after the negative pressure therapy. Three patients reported existing difficulties with cramps in the legs and two of the three (67%) reported improvements in the occurrence of the cramps after the negative pressure therapy, while the third did not apply the treatment according to the parameters of the disclosed methods. Five of the patients reported existing difficulties with sensibility disturbances, either reduced sensibility (described as numbness) or paresthesia (described as tingling, electrical shock, burning or prickling sensation) and four of the five patients reported improvements (80%) after the negative pressure therapy.

Four of the patients reported existing difficulties with a certain degree of pain in their legs and each of the four (100%) reported improvements after the negative pressure therapy. Three of the patients reported existing difficulties with fatigue as a significant symptom of their MS and two of the three reported improvements in fatigue levels (67%) after the negative pressure therapy. Four of the patients reported existing difficulties with sleep disturbances and poor sleep quality due to symptoms of MS manifesting in the legs, and each of the four (100%) reported improvements in sleep quality after the negative pressure therapy. Further, three of the patients reported an improvement in mood (42%), four of the patients reported an improvement in functionality (57%), and five of the patients reported an improvement in quality of life (71%). The reported improvements are clearly evidence of new and unexpected results from the treatment of MS patients through application of negative pressure therapy according to embodiments of the present disclosure.

The case studies above clearly demonstrate the advantages of treatment of spasticity using negative pressure therapy according to the current disclosure, including the prevention of spasticity and the treatment of ongoing spasticity. No similar advantages have been at all considered or realized in the prior art.

As is readily apparent from the foregoing discussion, it is understood that the duration, pressure, timing and location of negative pressure therapy and the associated devices can be adjusted so many different users having different body parts and varying levels of spasticity may benefit from the present methods. It is also understood that the negative pressure therapy according to the present disclosure can be adjusted for use in combination with other known treatments for spasticity.

## Claims

1. An anti-spasmodic composition for use in a method of treating or preventing spasticity in a patient, the method comprising:
administering a result effective dose of the anti-spasmodic composition; and
applying negative pressure therapy to the patient.

2. The anti-spasmodic composition for the use according to claim 1, wherein said administering step follows the detection of increased spasticity in the patient.

3. The anti-spasmodic composition for the use according to any of the preceding claims, wherein said negative pressure therapy comprises:
enclosing an area of skin of the patient in an enclosure such that the area of skin is sealed from external conditions; and
subjecting the area of skin to a negative pressure.

4. The anti-spasmodic composition for the use according to any of the preceding claims, wherein said negative pressure is between -20 mmHg and -80 mmHg.

5. The anti-spasmodic composition for the use according to any of the preceding claims, wherein said negative pressure is a pulsating pressure.

6. The anti-spasmodic composition for the use according to any of the preceding claims, wherein said pulsating pressure comprises generating a negative pressure against the area of skin for a first predetermined time interval and releasing the negative pressure for a second predetermined time interval.

7. The anti-spasmodic composition for the use according to any of the preceding claims, wherein the first predetermined time interval is different than the second predetermined time interval.

8. The anti-spasmodic composition for the use according to any of the preceding claims, wherein the first predetermined time interval and the second predetermined time interval each have a duration greater than a heartbeat of the patient.

9. The anti-spasmodic composition for the use according to any of the preceding claims, wherein the area of skin of the patient corresponds to the spasticity in the patient.

10. The anti-spasmodic composition for the use according to any of the preceding claims, wherein said applying negative pressure therapy to the patient comprises a treatment of at least 30 minutes, such as at least 40 minutes, such as at least 1 hour.

11. The anti-spasmodic composition for the use according to any of the preceding claims, wherein said applying negative pressure therapy to the patient comprises at least two treatments per day.

12. The anti-spasmodic composition for the use according to any of the preceding claims, wherein said applying step and/or said administration step is applied prior to the patient sleeping.

13. The anti-spasmodic composition for the use according to any of the preceding claims, wherein said applying step is performed for at least two limbs of the patient.

14. The anti-spasmodic composition for the use according to any of the preceding claims, wherein said applying step comprises applying an effective amount of negative pressure therapy to a limb of the patient,
wherein the limb is a limb affected by or nearest to an area of spasticity.

15. The anti-spasmodic composition for the use according to any of the preceding claims, wherein said spasticity is a result of brain injury, spinal cord injury, cerebral palsy, stroke, fibromyalgia, myalgic encephalomyelitis/chronic fatigue syndrome, neuromyelitis optica, Parkinson's disease, amyotrophic lateral sclerosis, multiple sclerosis (MS) or other neurodegenerative diseases.

16. The anti-spasmodic composition for the use according to any of the preceding claims, wherein the method comprises:
intermittent activation of a venous arterial reflex in the patient.

## Patentansprüche

1. Antispasmodische Zusammensetzung zur Verwendung bei einem Verfahren zur Behandlung oder Prävention von Spastik in einem Patienten, wobei das Verfahren umfasst:
Verabreichen einer im Ergebnis wirksamen Dosis der antispasmodischen Zusammensetzung; und
Anwenden einer Unterdrucktherapie bei dem Patienten.

2. Antispasmodische Zusammensetzung zur Verwendung gemäß Anspruch 1, wobei der Verabreichungsschritt auf die Feststellung einer erhöhten Spastik bei dem Patienten folgt.

3. Antispasmodische Zusammensetzung zur Verwendung gemäß einem der vorstehenden Ansprüche, wobei die Unterdrucktherapie umfasst:
Umschließen eines Bereichs der Haut des Patienten mit einer Umhüllung, sodass der Bereich der Haut gegenüber äußeren Bedingungen abgedichtet ist; und
Aufbringen eines Unterdrucks auf den Bereich der Haut.

4. Antispasmodische Zusammensetzung zur Verwendung gemäß einem der vorstehenden Ansprüche, wobei der Unterdruck zwischen -20 mm Hg und -80 mm Hg liegt.

5. Antispasmodische Zusammensetzung zur Verwendung gemäß einem der vorstehenden Ansprüche, wobei der Unterdruck ein pulsierender Druck ist.

6. Antispasmodische Zusammensetzung zur Verwendung gemäß einem der vorstehenden Ansprüche, wobei der pulsierende Druck das Erzeugen eines Unterdrucks an dem Bereich der Haut in einem ersten vorbestimmten Zeitintervall und das Aufheben des Unterdrucks in einem zweiten vorbestimmten Zeitintervall umfasst.

7. Antispasmodische Zusammensetzung zur Verwendung gemäß einem der vorstehenden Ansprüche, wobei das erste vorbestimmte Zeitintervall von dem zweiten vorbestimmten Zeitintervall unterschiedlich ist.

8. Antispasmodische Zusammensetzung zur Verwendung gemäß einem der vorstehenden Ansprüche, wobei das erste vorbestimmte Zeitintervall und das zweite vorbestimmte Zeitintervall jeweils eine längere Dauer als eine Herzaktion des Patienten aufweisen.

9. Antispasmodische Zusammensetzung zur Verwendung gemäß einem der vorstehenden Ansprüche, wobei der Bereich der Haut des Patienten der Spastik bei dem Patienten entspricht.

10. Antispasmodische Zusammensetzung zur Verwendung gemäß einem der vorstehenden Ansprüche, wobei das Anwenden einer Unterdrucktherapie bei dem Patienten eine Behandlung von mindestens 30 Minuten, wie zum Beispiel von mindestens 40 Minuten, wie zum Beispiel von mindestens 1 Stunde umfasst.

11. Antispasmodische Zusammensetzung zur Verwendung gemäß einem der vorstehenden Ansprüche, wobei das Anwenden einer Unterdrucktherapie bei dem Patienten mindestens zwei Behandlungen pro Tag umfasst.

12. Antispasmodische Zusammensetzung zur Verwendung gemäß einem der vorstehenden Ansprüche, wobei der Schritt des Anwendens und/oder der Schritt des Verabreichens vor der Schlafperiode des Patienten durchgeführt werden.

13. Antispasmodische Zusammensetzung zur Verwendung gemäß einem der vorstehenden Ansprüche, wobei der Schritt des Anwendens an mindestens zwei Körpergliedern des Patienten durchgeführt wird.

14. Antispasmodische Zusammensetzung zur Verwendung gemäß einem der vorstehenden Ansprüche, wobei der Schritt des Anwendens das Anwenden eines wirksamen Maßes der Unterdrucktherapie an einem Körperglied des Patienten umfasst,
wobei das Körperglied ein Körperglied ist, das durch oder am nächsten zu einem Bereich der Spastik betroffen ist.

15. Antispasmodische Zusammensetzung zur Verwendung gemäß einem der vorstehenden Ansprüche, wobei die Spastik ein Ergebnis einer Hirnverletzung, einer Rückenmarksverletzung, einer Zerebralparese, eines Schlaganfalls, einer Fibromyalgie, einer myalgischen Enzephalomyelitis/eines chronischen Ermüdungssyndroms, einer Neuromyelitis Optica, einer Parkinson-Krankheit, einer amyotrophen Lateralsklerose, einer multiplen Sklerose (MS) oder von anderen neurodegenerativen Krankheiten ist.

16. Antispasmodische Zusammensetzung zur Verwendung gemäß einem der vorstehenden Ansprüche, wobei das Verfahren umfasst:
intermittierende Aktivierung eines veno-arteriolären Reflexes in dem Patienten.

## Revendications

1. Composition anti-spasmodique destinée à être utilisée dans le traitement ou la prévention de la spasticité chez un patient, le procédé comprenant :
l'administration d'une dose efficace résultante de la composition anti-spasmodique ; et
l'application d'une thérapie de pression négative au patient.

2. Composition anti-spasmodique destinée à être utilisée selon la revendication 1, dans laquelle l'étape d'administration suit la détection d'une spasticité accrue chez le patient.

3. Composition anti-spasmodique destinée à être utilisée selon l'une quelconque des revendications précédentes, dans laquelle la thérapie de pression négative comprend :
l'enfermement d'une zone de peau du patient dans une clôture de telle manière que la zone de peau est scellée des conditions externes et
la soumission de la zone de peau à une pression négative.

4. Composition anti-spasmodique destinée à être utilisée selon l'une quelconque des revendications précédentes, dans laquelle ladite pression négative est comprise entre -20 mm Hg et -80 mm Hg.

5. Composition anti-spasmodique destinée à être utilisée selon l'une quelconque des revendications précédentes, dans laquelle ladite pression négative est une pression de pulsation.

6. Composition anti-spasmodique destinée à être utilisée selon l'une quelconque des revendications précédentes, dans laquelle ladite pression de pulsation comprend la production d'une pression négative contre la zone de peau pour un premier intervalle de temps prédéterminé et la libération de la pression négative pour un second intervalle de temps prédéterminé.

7. Composition anti-spasmodique destinée à être utilisée selon l'une quelconque des revendications précédentes, dans laquelle le premier intervalle de temps prédéterminé est différent du second intervalle de temps prédéterminé.

8. Composition anti-spasmodique destinée à être utilisée selon l'une quelconque des revendications précédentes, dans laquelle le premier intervalle de temps prédéterminé et le second intervalle de temps prédéterminé ont chacun une durée plus grande qu'un battement de cœur du patient.

9. Composition anti-spasmodique destinée à être utilisée selon l'une quelconque des revendications précédentes, dans laquelle la zone de peau du patient correspond à la spasticité chez le patient.

10. Composition anti-spasmodique destinée à être utilisée selon l'une quelconque des revendications précédentes, dans laquelle ladite application de thérapie de pression négative au patient comprend un traitement d'au moins 30 minutes, tel que d'au moins 40 minutes, tel que d'au moins 1 heure.

11. Composition anti-spasmodique destinée à être utilisée selon l'une quelconque des revendications précédentes, dans laquelle ladite application de thérapie de pression négative au patient comprend au moins deux traitements par jour.

12. Composition anti-spasmodique destinée à être utilisée selon l'une quelconque des revendications précédentes, dans laquelle ladite étape d'application et/ou ladite étape d'administration est appliquée avant le sommeil du patient.

13. Composition anti-spasmodique destinée à être utilisée selon l'une quelconque des revendications précédentes, dans laquelle ladite étape d'application est effectuée pour au moins deux membres du patient.

14. Composition anti-spasmodique destinée à être utilisée selon l'une quelconque des revendications précédentes, dans laquelle ladite étape d'application comprend l'application d'une quantité efficace de thérapie de pression négative à un membre du patient,
le membre étant un membre affecté par ou le plus proche à une zone de spasticité.

15. Composition anti-spasmodique destinée à être utilisée selon l'une quelconque des revendications précédentes, dans laquelle ladite spasticité est un résultat de lésion cérébrale, de lésion de la moelle épinière, de paralysie cérébrale, d'accident vasculaire cérébral, de fibromyalgie, d'encéphalomyélite myalgique / syndrome de fatigue chronique, de l'optica de neuromyélite, de la maladie de Parkinson, de la sclérose latérale amyotrophique, de la sclérose multiple (MS) ou d'autres maladies neurodégénératives.

16. Composition anti-spasmodique destinée à être utilisée selon l'une quelconque des revendications précédentes, dans laquelle le procédé comprend :
l'activation intermittente d'un réflexe artériel veineux chez le patient.
